# EUROPÄISCHE PATENTSCHRIFT

(11) **EP 1 135 203 B1**
(45) Veröffentlichungstag und Bekanntmachung des Hinweises auf die Patenterteilung: **05.06.2002**
(21) Anmeldenummer: 99953912.5
(22) Anmeldetag: 21.10.1999
(51) Int. Cl.: B01J 8/00, B01J 8/24

(54) **VORRICHTUNG ZUR BEREITSTELLUNG EINES BLOCKFLANSCHES EINER MANNLOCHÖFFNUNG OD. DGL., INSBESONDERE BEI WIRBELSCHICHTREAKTOREN**
DEVICE FOR PROVIDING A LOOSE FLANGE OF A MANHOLE OPENING OR THE LIKE, ESPECIALLY FOR FLUIDIZED REACTORS
DISPOSITIF POUR PREPARER LA BRIDE FOLLE D'UN TROU D'HOMME OU SIMILAIRE, NOTAMMENT POUR DES REACTEURS A LIT FLUIDISE

(30) Priorität: 07.11.1998 DE 19851423
(43) Veröffentlichungstag der Anmeldung: 26.09.2001
(73) Patentinhaber: Krupp Uhde GmbH, 44141 Dortmund (DE)
(72) Erfinder: SCHÖN, Hartmut, D-61440 Oberursel (DE)
(74) Vertreter: Patentanwälte Meinke, Dabringhaus und Partner
(86) Internationale Anmeldenummer: EP9908001
(87) Internationale Veröffentlichungsnummer: WO0027517

(56) Entgegenhaltungen:
- WO-A-93/11863
- US-A- 4 074 691

## Beschreibung

Die Erfindung richtet sich auf einen Blockflansch einer Mannlochöffnung od. dgl. für Wirbelschichtreaktoren zur Oxichiorierung von Ethylen, Sauerstoff und HCl mit einem in der Reaktorwand befestigten Wandflansch.

Bei der sogenannten Oxichlorierung werden Ethylen, Sauerstoff und HCl in einem Wirbelschichtreaktor (Oxireaktor) an einem kupferhaltigen Katalysator zu 1,2-Dichlorethan und Wasser umgesetzt. Innerhalb des Reaktors können bei Betrieb und bei Abstellen der Anlage Katalysatorablagerungen in allen Toträumen auftreten. Das Vorhandensein von HCl führt bei der Taupunktunterschreitung zur Salzsäurebildung und damit zu heftiger Korrosion. Besonders gefährdet sind dabei Toträume im Mantelbereich, wie alle Mannlöcher und alle Meßstutzen. Man hat diesem Nachteil versucht, dadurch abzuhelfen, daß beispielsweise bei allen Meßstutzen beheizte Spülleitungen vorgesehen sind, um auch eine Beeinträchtigung der Messungen und der Taupunktunterschreitungen dort zu vermeiden.

Bei den Mannlochgestaltungen ist es bekannt, die Deckel dieser Mannlöcher nach innen mit einem zusätzlichen Verdrängungskörper auszurüsten, der so gestaltet ist, daß möglichst ein glatter fluchtender Übergang mit der Reaktormantelinnenfläche gegeben ist.

Aufgabe der Erfindung ist die Schaffung einer Lösung, mit der alle diese zusätzlichen Hilfsmaßnahmen entbehrlich sind und der damit verbundene Aufwand eingespart werden kann, ohne daß die Funktionsfähigkeit des Systems darunter leidet und ohne daß die nicht gewünschten Ablagerungen in diesen Bereichen auftreten.

Mit einer Vorrichtung der eingangs bezeichneten Art wird diese Aufgabe gemäß der Erfindung dadurch gelöst, daß die in das Reaktorinnere und in Schwerkraftrichtung untere Flanschfläche wenigstens bereichsweise angeschrägt ausgebildet ist.

Mit der Erfindung sind eine Reihe von Vorteilen verbunden, so werden mögliche Toträume, die in den Katalysatorablagerungen vorkommen können, verringert und alle beheizten Spülleitungen, die bei den bekannten Lösungen eingesetzt werden, mit Filterstationen, Durchflußmessern, Kondensatorsammlern, Halterungen u. dgl. entfallen.

Besonders vorteilhaft ist es, wenn, wie dies die Erfindung bei einem ringförmigen Flansch ebenfalls vorsieht, die Abschrägung der Fläche von der horizontalen Mittelebene im Innenrandbereich beginnend zur vertikalen Mitte des Flanschringes sich vergrößernd ausgebildet ist.

Mit dieser Gestaltung ergibt sich eine glatte homogene, trichterförmige Rutsche, die nach innen zum Reaktor weist, so daß beispielsweise beim Abschalten und Absinken der Wirbelschicht eine Ablagerung von Katalysatormaterial od. dgl. mit Sicherheit verhindert wird.

Weitere Merkmale, Einzelheiten und Vorteile der Erfindung ergeben sich aufgrund der nachfolgenden Beschreibung sowie anhand der Zeichnung. Diese zeigt in
- Fig. 1: eine vereinfachte räumliche Darstellung eines Blockflansches nach der Erfindung,
- Fig. 2: eine vereinfachte Schnittdarstellung eines Mannloches sowie in
- Fig. 3: eine vereinfachte Schnittdarstellung eines Blockflansches etwa gemäß Fig. 1.

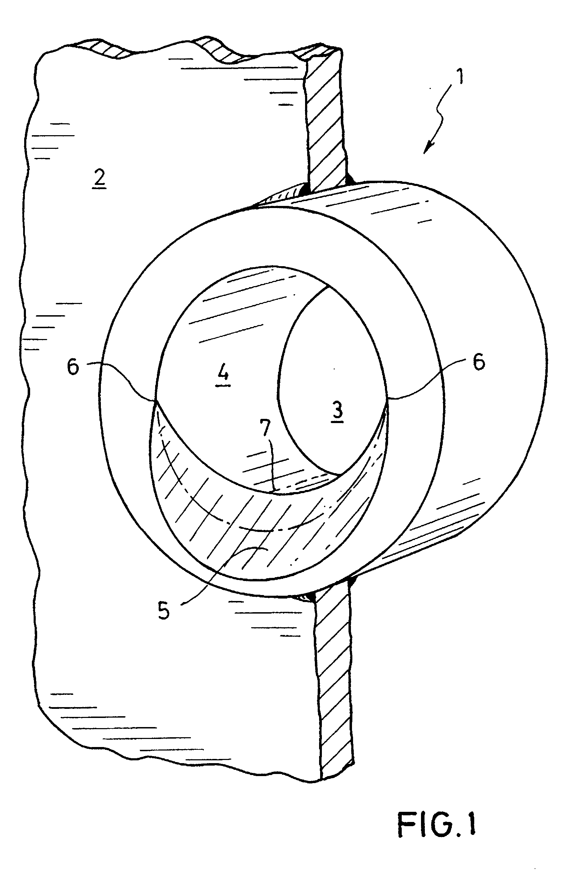

Bezug nehmend auf Fig. 1 ist ein allgemein mit 1 bezeichneter Blockflansch nach der Erfindung in einer Reaktorwand 2 eingeschweißt. Der Blockflansch hat eine gewisse zylindrische Erstreckung und weist eine Durchtrittsöffnung 3, z.B. für ein Meßgerät, eine Sonde od. dgl., auf.

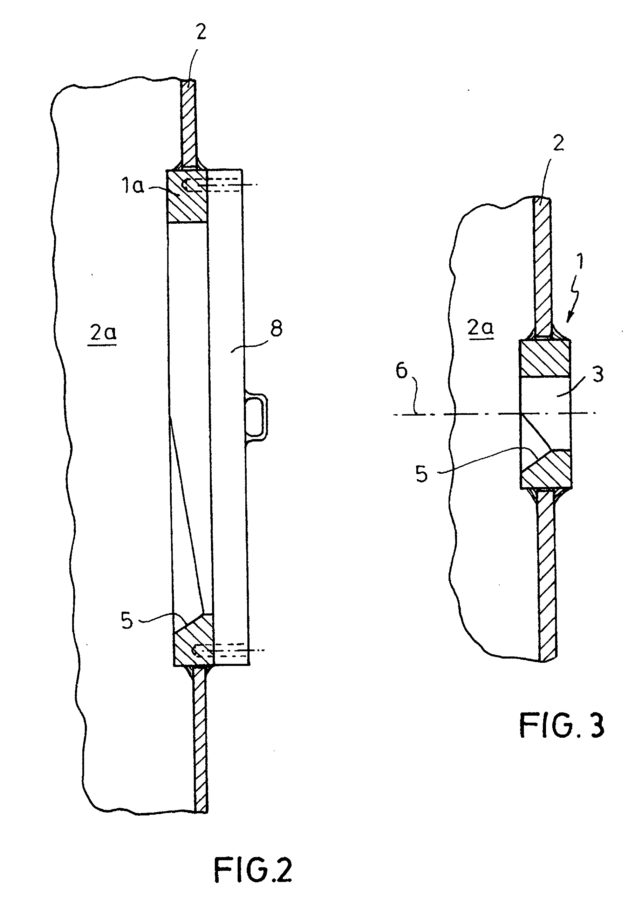

In seinem zum Inneren, in Fig. 2 mit 2a bezeichnet, weisenden Bereich ist die Flanschinnenfläche 4 abgeschrägt, die Abschrägung ist in Fig. 1 mit 5 bezeichnet. Diese abgeschrägte Fläche 5 beginnt etwa in der Mittelhorizontalen 6 des Flansches 1, verläuft bis in die horizontale Mitte, mit 7 bezeichnet, des Flansches und von dort wieder bis zur vertikalen Mitte 6, so daß sich eine Art Schütte ergibt. Dort auffallendes Material wird damit automatisch aufgrund der Schwerkraft abgeworfen.

Fig. 2 zeit eine Mannlochausbildung, auch hier ist eine entsprechende Flanschgestaltung vorgesehen, das Mannloch ist mit einem dort mit 8 bezeichneten Deckel verschlossen.

In Fig. 3 ist ein derartiger Blockflansch 1 im Schnitt dargestellt.

Natürlich sind die beschriebenen Ausführungsbeispiele der Erfindung noch in vielfacher Hinsicht abzuändern, ohne den Grundgedanken zu verlassen. So können die Flansche 1 bzw. 1a insbesondere mit der Innenwandfläche der Reaktorwand 2 fluchtend eingeschweißt sein, der Flansch bzw. die Flanschbohrung 3 kann in einem nach unten weisenden geringfügigen Winkel angeordnet sein, um das Abrieseln auffallenden Materiales zu verstärken u. dgl. mehr.

## Patentansprüche

1. Blockflansch einer Mannlochöffnung od. dgl. für Wirbelschichtreaktoren zur Oxichlorierung von Ethylen, Sauerstoff und HCl mit einem in der vertikalen Reaktorwand befestigten Wandflansch,
**dadurch gekennzeichnet,**
**daß** die in das Reaktorinnere und in Schwerkraftrichtung untere Flanschfläche (5) wenigstens bereichsweise angeschrägt ausgebildet ist.

2. Blockflansch nach Anspruch 1, wobei der Flansch ringförmig ausgebildet ist,
**dadurch gekennzeichnet,**
**daß** die Abschrägung der Fläche (5) von der horizontalen Mittelebene (6) im Innenrandbereich beginnend zur vertikalen Mitte (7) des Flanschringes sich vergrößernd ausgebildet ist.

## Claims

1. Loose flange on a manhole opening or the like designed for fluidised bed reactors for the oxychlorination of ethylene, oxygen and HCl, having a wall flange fixed in the vertical wall of the reactor,
**characterised in that** the lower flange surface (5) into the reactor's interior and in the direction of gravity is constructed with a slope in at least some places.

2. Loose flange according to claim 1, where the flange is a ring-shaped construction,
**characterised in that** the slope of the surface (5) increases from the horizontal central plane (6) in the inner peripheral zone to the vertical centre (7) of the flange ring.

## Revendications

1. Bride-bloc pour un trou de visite ou similaire pour des réacteurs à lit fluidisé pour l'oxychloration d'éthylène, d'oxygène et de HCl, comportant une bride de paroi fixée dans la paroi verticale du réacteur, **caractérisée en ce que** la surface de bride (5) inférieure dans l'intérieur du réacteur et en direction de gravité est réalisée du moins localement biseautée.

2. Bride-bloc selon la revendication 1, la bride étant réalisée en forme annulaire, **caractérisée en ce que** le biseautage de la surface (5) est réalisé de manière à s'agrandir à partir du plan central horizontal (6) jusqu'au milieu vertical (7) de l'anneau de bride, en commençant dans la zone de bordure intérieure.
